# EUROPEAN PATENT SPECIFICATION

(11) **EP 1 267 757 B1**
(45) Date of publication and mention of the grant of the patent: **03.11.2004**
(21) Application number: 01914044.1
(22) Date of filing: 23.03.2001
(51) Int. Cl.: A61F 5/00

(54) **DEVICE FOR RELIEF OF ANAL PAIN**
EINRICHTUNG ZUR VERMINDERUNG VON ANALSCHMERZ
DISPOSITIF PERMETTANT DE SOULAGER UNE DOULEUR ANALE

(30) Priority: 24.03.2000 GB 0007145; 16.10.2000 US 690693
(43) Date of publication of application: 02.01.2003
(73) Proprietor: Behive Limited, Basingstoke, Hampshire RG26 3TN (GB)
(72) Inventor: HOLLAND, Graham, Michael, Basingstoke,Hampshire RG24 9EH (GB)
(74) Representative: Wolff, Francis Paul
(86) International application number: PCT/GB2001/001292
(87) International publication number: WO 2001/072247

(56) References cited:
- DE-C- 648 690
- US-A- 1 482 077
- US-A- 3 826 242
- US-A- 4 563 182
- US-A- 4 841 970

## Description

This invention relates to a device for relief of pain. More specifically, it relates to a device for the relief of anal pain arising from hemorrhoids (piles) or other anal problems. A device as defined in the preamble of claim 1 is disclosed in DE-C- 648 690.

A number of devices have been provided in the art for relief of anal pain, but most of them are not suitable for contingent use by the sufferer himself, or are not able to provide relief inexpensively.

EP-A-0 672 400 describes a cooling cylindrical device for removal of pain and therapeutic treatment of hemorrhoids and anal fissures comprising a hollow insert, finger or bulb shaped for insertion into the anus with at least one inlet and/or outlet openings, and at least one container for cold liquid circulation starting from a container through an inlet tube into the cylindrical insert, the liquid being driven back through the outlet opening and through the second tube to the container, under the influence of a pump connected to the container or back to another container under the influence of gravity.

US-A-4 331 151 describes a hemorrhoid bandage or cold pack, for positioning within a human anal canal, formed of a hollow, thin wall, roughly cylindrically shaped body having an inner end and an outer end. The body is transversely divided into two parts by a central panel extending longitudinally from the outer end towards the inner end, but having an open area adjacent the inner end. A tube, which is coaxial with the body, extends from the inner to the outer end and opens exteriorly of the body at each end. A fluid inlet continuously supplies fluid having a predetermined temperature into one body part through the outer end, which fluid flows through the panel open area at the inner end, and then out of the other body part through a fluid drain opening communicating therewith. The two interior parts may be further subdivided by transverse ribs extending between the panel and the body exterior wall to form longitudinally extending channels for controlling the direction of the fluid flow through the body. By using a relatively cool fluid, such as cool water, the bandage may be used to provide a controlled temperature, for extended periods of time, within the rectal area in connection with treatment of hemorrhoids.

US-A-4 563 182 describes a method of treating hemorrhoids which comprises inserting into the rectum of a subject afflicted therewith a substantially cylindrical shaped insert, comprising a water swellable polymer having a water content of at least 35% by weight, the insert having previously been subjected to a temperature below 0°C for a sufficient amount of time to freeze the free water therein, and maintaining the inset with at least a portion thereof outside the sphincter muscle.

US-A-4 841 970 describes a cryogenic proctologic insert for treating hemorrhoids by lowering the surface temperature of the affected portion of the rectal canal. The insert is formed of a tubular plastic portion filled with a congealable fluid. Extended heat transfer surfaces are provided in the interior of the insert to promote heat transfer to and from the fluid.

US-A-4 537 194 relates to an applicator used to apply a frozen solid, such as ice, to a wound or injury to treat the wound or injury. A first container has an open top of a predetermined size, a tapered side wall and a bottom having a central opening therethrough, and a second container has a shape conforming at least in part to that of the first container, an open top and a closed bottom. The two containers are fitted together by placing the bottom of the first container into the top of the second container. The device can then be filled with liquid and frozen until needed. When in use as an applicator, the second container is removed and the first container serves as a handle for applying the frozen solid.

DE-A-3 802 218 relates to a method and a device for producing ice for medical treatment, especially for therapeutic massage, in which water filled in a container is subjected to a freezing process. During the freezing process the water present in the container is mou lded onto a carrier element as a compact body of ice which is uncovered during the medical treatment

Other devices are known of greater or lesser complication and practicality. There still remains the need, however, for a simple and inexpensive means of providing relief for anal pain.

In accordance with the present invention there is provided a device for relief of anal pain comprising a rod having first and second opposite end segments, the first end segment being shaped for grasping in the hand, a substantially annular flange structure between the first and second end segments, the flange structure having an external cylindrical surface, and a cap having an interior surface defining a cavity for receiving the second end segment of the rod, the interior surface of the cap being adapted to engage in sealing fashion the external cylindrical surface of the flange.

For a better understanding of the invention, and to show how the same may be carried into effect, reference will now be made, by way of example, to the accompanying drawings, wherein:
Figure 1 represents a perspective view of one embodiment of a device according to the invention;
Figure 2 represents a section through the device; and
Figure 3 is a partial sectional view of another embodiment of a device in accordance with the invention.

Referring now to the drawings, the device according to a first embodiment of the invention provides a rod having a first portion 1 adapted to fit within the hand. At one end of this portion, the rod is optionally provided with an enlarged portion 2. This is shown as having a shape that is substantially a disc, but other shapes can be employed, the purpose of the enlarged portion being for convenient holding in the hand. At its other end, the rod 1 has a shaped end over which fits a cap 3. The fit is intended to be a sealing fit over the rod, in a manner to be described, but easily removable by simple pulling.

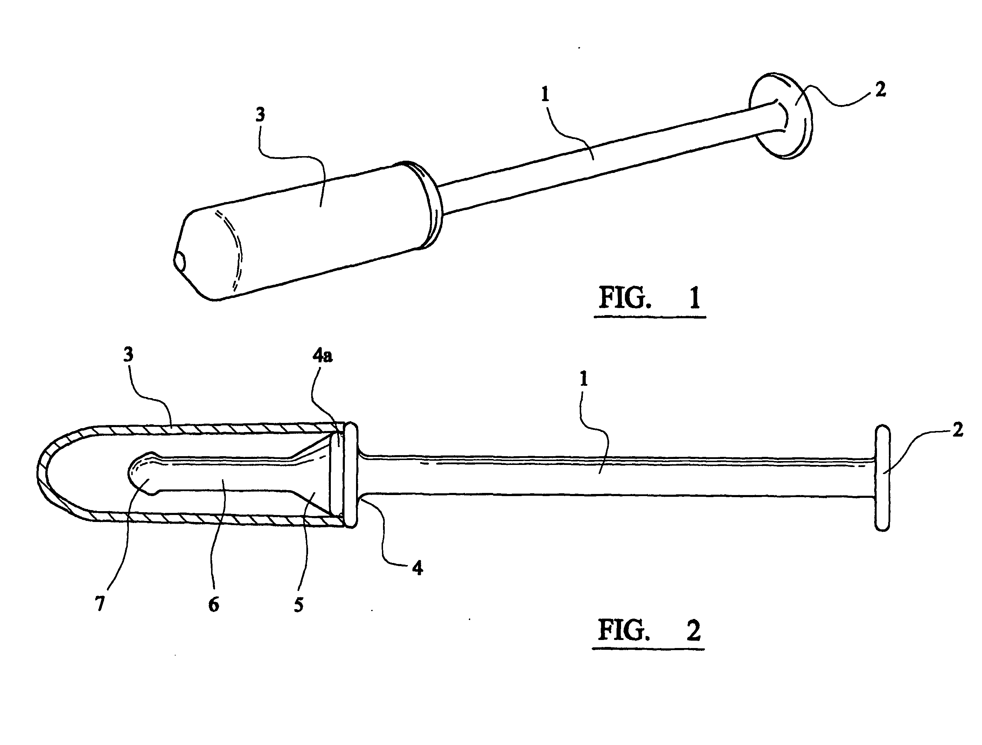

As will be seen more clearly from Fig 2, the shaped end of the rod adapted to fit within the cap 3 lies beyond a flange 4 of greater diameter than the first portion of the rod. The portion of the rod within the cap is formed as a cylindrical shoulder portion 4a protruding from flange 4, but of lesser diameter; a substantially conical portion 5 whose diameter diminishes from shoulder 4a, down towards the end of the rod; a further substantially cylindrical portion 6; and a bulb 7 at the end. The exact shapes of the bulb and other elements are not material to the invention, provided that they are of such a form as not to cause tissue damage. The cap 3 fits over the end of the rod and constitutes a push fit over cylindrical shoulder 4a, so as to form a seal over the rod end and fit flush with the flange 4.

The dimensions of the device according to the invention are not critical, subject to the overriding factor that they must not be too small or too large for the device to fulfill its intended function.

Typically the rod 1 may have an overall length i.e. the length from the enlarged portion 2 to the top of bulb 7 of from 110 to 130 mm, preferably about 120 mm. The first portion 1 may have a length from 75 to 85 mm, preferably 50 mm, and a diameter from 4 to 6 mm, preferably 5 mm. The total length from the base of the conical portion 5 to the tip of the bulb 7 may be from 30 to 40 mm, preferably 35 mm, and the diameter of portion 6 may be from 3.5 to 4.5 mm, preferably 4 mm. The two wider portions 2 and 4 may have thicknesses of 1.5 to 2.5 mm, preferably 2 mm, and diameters from 12 to 16 mm, preferably 14 mm, in the case of portion 2, and 15 mm, in the case of portion 4. The cap 3 may have a length of 40 to 60 mm, preferably 50 mm, an external diameter of 13 to 17 mm, preferably 15 mm, and an internal diameter of 11 to 14 mm, preferably 12.5 mm.

The space between the cap and the end of the rod can be filled with water, optionally containing a disinfectant or analgesic material, and this water can then be frozen.

For use, the cap is removed from the rod, and the ice, substantially in the form of a cylinder or tapered cylinder with an end again forming a slightly rounded conical portion, can then be inserted into the anus for the contingent relief of pain.

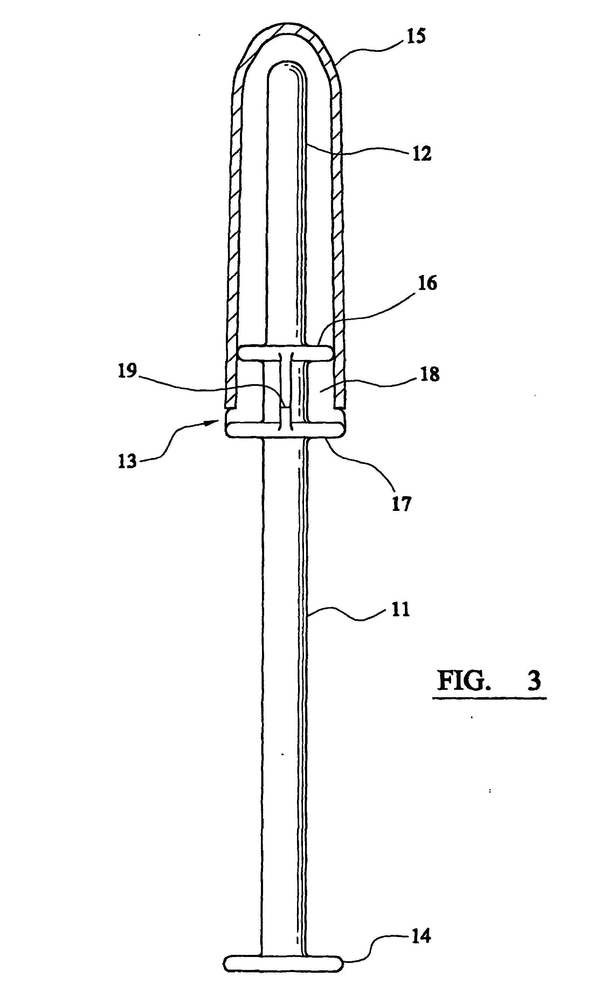

Referring now Figure 3, a further embodiment of the device according to the invention comprises a rod having first and second opposite end segments 11 and 12 extending to opposite respective sides of a flange structure 13. The end segment 11 is adapted to be grasped in the hand. At the free end of this segment, the rod is optionally provided with an enlarged portion 14 for convenient holding in the hand. The enlarged portion 14 is shown as having a shape that is substantially a disc but, as with the previously-described embodiment, other shapes can be employed.

The flange structure 13 includes a smaller diameter annular flange 16, a larger diameter annular flange 17, and four linear webs 18 extending between the flanges 16 and 17. The webs 18 are each formed with a step 19 to provide a transition from the smaller diameter of the flange 16 to the larger diameter of the flange 17.

A cap 15, having generally the same form as the cap 3 of the first embodiment, fits over the second end segment 12 of the rod. The cap 15 fits over the end segment 12 with a push fit, so as to form a seal with the smaller flange 16. Nevertheless, the cap can be easily removable by simple pulling. The rim of the cap 15 engages the step 19, which ensures a clearance between the rim of the cap and the larger flange 17.

The dimensions of the device according to the invention are not critical, subject to the overriding factor that they must not be too small or too large for the device to fulfill its intended function. Typically the rod may have an overall length of from 110 to 130 mm, preferably about 120 mm. The first end segment 11 may have a length from 75 to 85 mm, preferably 80 mm, and a diameter from 4 to 6 mm, preferably 5 mm. The second end segment 12 may have a length from 30 to 40 mm, preferable about 35 mm, and the diameter of the end segment 12 may be from 3.5 to 4.5 mm, preferably 4 mm. The enlarged portion 14 and the larger flange 17 each may have a thickness of 1.5 to 2.5 mm, preferably 2 mm, and diameter from 12 to 16 mm, preferably 14 mm. The smaller flange 16 may have a thickness of 1.5 to 2.5 mm, preferably 2 mm, and a diameter from 11.5 to 13.5 mm, preferably 12.5 mm. The cap 15 may have a length of 40 to 60 mm, preferably 50 mm and an external diameter of 13 to 17 mm, preferably 15 mm

To prepare for use, the cap 15 is filled with water, optionally containing a disinfectant or analgesic material, and the end segment 12 is inserted fully in the cap so that the smaller flange 16 enters the cap and the step 19 abuts the rim of the cap. The interior surface of the cap seals against the exterior of the smaller flange so that the water is retained in the cap. The device is then placed in a freezer for sufficient time for the water to freeze. Expansion of the water when it freezes is accommodated by the sliding fit ofthe cap 15 on the smaller flange 16. For use, the cap is removed from the rod and a body of ice substantially in the form of a cylinder or tapered cylinder, with an end again forming a slightly rounded conical portion, is held firmly on the end segment 12. The user can then grip the device by the end segment 11, with the aid of the enlarged portion 14, and insert the ice body into the anus for the contingent relief of pain.

The webs 18 ensure that the end segment 12 is aligned with the central axis of the cap when the cap is fitted on the end segment 12. The step 19 provided on the webs 18 limits the depth to which the end segment 12 can enter the cap and therefore ensures that the tip of the end segment is covered by the end of the ice body.

It will be appreciated that the invention is not restricted to the particular embodiment that has been described, and that variations may be made therein without departing from the scope of the invention as defined in the appended claims and equivalence thereof.

## Claims

1. A device for relief of anal pain comprising:
a rod (1) having first and second opposite end segments, the first end segment being shaped for grasping in the hand,
a substantially annular flange structure (4) between the first and second end segments, **characterised in that** the flange structure has an external cylindrical surface (4a), and **in that** it comprises a cap (3) having an interior surface defining a cavity for receiving the second end segment of the rod, the interior surface of the cap being adapted to engage in sealing fashion the external cylindrical surface (4a) of the flange.

2. A device according to claim 1, wherein the cap has a rim and the flange structure includes a shoulder which is abutted by the rim of the cap when the cap is fitted over the second end segment of the rod to limit penetration of the second end segment into the interior of the cap.

3. A device according to claim 1, wherein the flange structure includes an annular flange having an external peripheral surface that constitutes said external cylindrical surface.

4. A device according to claim 1, wherein the flange structure includes a smaller annular flange (16), and a plurality of linear webs connecting the smaller (16) and larger (17) flanges, and wherein the smaller annular flange is between the larger annular flange and the second end segment of the rod and the smaller annular has a external peripheral surface that constitutes said external cylindrical surface.

5. A device according to claim 4, wherein the webs include a step between the smaller annular flange and the larger annular flange and the step is abutted by the rim of the cap when the cap is fitted over the second end segment of the rod to limit penetration of the second end segment into the interior of the cap.

6. A device according to claim 1, wherein the flange structure constitutes the base of a conical portion whose diameter diminishes towards the tip of the end segment remote from the enlarged portion.

7. A device according to claim 6 wherein the tip of the end segment remote from the enlarged portion is provided with a bulb (7).

## Patentansprüche

1. Ein Gerät zur Verminderung von Analschmerz, enthaltend:
einen Stab (1), der erste und zweite gegenüberliegende Endsgemente hat, wobei das erste Endsegment eine Form zum Festhalten in der Hand hat,
eine im wesentlichen ringförmige Flanschstruktur (4) zwischen den ersten und zweiten Endsegmenten, **dadurch gekennzeichnet, dass** die Flanschstruktur eine äußere Zylinderfläche (4 a) aufweist, und dass es
eine Kappe (3) enthält, die eine Innenfläche hat, welche einen Hohlraum zur Aufnahme des zweiten Endsegments des Stabs begrenzt, wobei die Innenfläche der Kappe so geformt ist, um in abdichtender Weise gegen die äußere Zylinderfläche (4 a) anzuliegen.

2. Ein Gerät nach Anspruch 1, bei dem die Kappe einen Rand hat und die Flanschstruktur eine Schulter enthält, gegen die der Rand der Kappe anliegt, wenn die Kappe über den zweiten Endabschnitt des Stabs gezogen ist, um das Eindringen des zweiten Endabschnitts in das Innere der Kappe zu begrenzen.

3. Ein Gerät nach Anspruch 1, bei dem die Flanschstruktur einen Ringflansch umfasst, der eine äußere Umfangsfläche hat, die diese äußere Zylinderfläche bildet.

4. Ein Gerät nach Anspruch 1, bei dem die Flanschstruktur einen kleineren Ringflansch (16) und mehrere lineare Stege umfasst, die die kleineren (16) und größeren (17) Flansche miteinander verbinden, und bei dem der kleinere Ringflansch zwischen dem größeren Ringflansch und dem zweiten Endsegment des Stabes liegt und der kleinere Ringflansch eine äußere Umfangsfläche hat, die diese äußere Zylinderfläche bildet.

5. Ein Gerät nach Anspruch 4, bei dem die Stege eine Abstufung zwischen dem kleineren Ringflansch und dem größeren Ringflansch enthalten, um bei dem die Abstufung gegen den Rand der Kappe stößt, wenn die Kappe über das zweite Endsegment des Stabs gezogen wird, um das Eindringen des zweiten Endsegments in das Innere der Kappe zu begrenzen.

6. Ein Gerät nach Anspruch 1, bei dem die Flanschstruktur die Basis eines Konusabschnitts bildet, dessen Durchmesser in Richtung der von dem größeren Abschnitt entfernt liegenden Spitze des Endsegments kleiner wird.

7. Ein Gerät nach Anspruch 6, bei dem die von dem größeren Abschnitt entfernt liegende Spitze des Endsegments mit einer Wulst (7) versehen ist.

## Revendications

1. Dispositif destiné à soulager une douleur anale, comprenant :
une tige (1) ayant des premier et second segments d'extrémité opposés, le premier segment d'extrémité étant formé pour être saisi dans la main,
une structure de collerette sensiblement annulaire (4) entre les premier et second segments d'extrémité, **caractérisée en ce que** la structure de collerette a une surface externe cylindrique (4a) et **en ce qu'**elle comprend un capuchon (3) ayant une surface intérieure définissant une cavité destinée à recevoir le second segment d'extrémité de la tige, la surface intérieure du capuchon étant adaptée pour engager de manière étanche la surface externe cylindrique (4a) de la collerette.

2. Dispositif selon la revendication 1, dans lequel le capuchon a un rebord et la structure de collerette comprend un épaulement contre lequel bute le rebord du capuchon lorsque le capuchon est adapté sur le second segment d'extrémité de la tige pour limiter la pénétration du second segment à l'intérieur du capuchon.

3. Dispositif selon la revendication 1, dans lequel la structure de collerette comprend une collerette annulaire ayant une surface périphérique externe qui constitue ladite surface externe cylindrique.

4. Dispositif selon la revendication 1, dans lequel la structure de collerette comprend une collerette annulaire plus petite (16) et une pluralité d'âmes linéaires connectant les collerettes plus petite (16) et plus grande (17), et dans lequel la collerette annulaire plus petite se trouve entre la collerette annulaire plus grande et le second segment d'extrémité de la tige et la collerette annulaire plus petite a une surface périphérique externe qui constitue ladite surface externe cylindrique.

5. Dispositif selon la revendication 4, dans lequel les âmes comprennent une marche entre la collerette annulaire plus petite et la collerette annulaire plus grande et dans lequel le rebord du capuchon bute contre la marche lorsque le capuchon est adapté sur le second segment d'extrémité de la tige pour limiter la pénétration du second segment d'extrémité à l'intérieur du capuchon.

6. Dispositif selon la revendication 1, dans lequel la structure de collerette constitue la base d'une partie conique dont le diamètre diminue vers le bout du segment d'extrémité distant de la partie agrandie.

7. Dispositif selon la revendication 6, dans lequel le bout du segment d'extrémité distant de la partie agrandie est muni d'un bulbe (7).
